**Europäisches Patentamt**

**European Patent Office**

**Office européen des brevets**

(19)

(11) Veröffentlichungsnummer : **0 516 672 B1**

(12) # EUROPÄISCHE PATENTSCHRIFT

(45) Veröffentlichungstag der Patentschrift :
**11.05.94 Patentblatt 94/19**

(51) Int. Cl.$^5$ : **C07K 1/04**

(21) Anmeldenummer : **91904242.4**

(22) Anmeldetag : **20.02.91**

(86) Internationale Anmeldenummer :
**PCT/EP91/00318**

(87) Internationale Veröffentlichungsnummer :
**WO 91/13084 05.09.91 Gazette 91/21**

(54) **VERFAHREN UND VORRICHTUNG ZUR SIMULTANEN SYNTHESE MEHRERER POLYPEPTIDE.**

(30) Priorität : **22.02.90 DE 4005518**

(43) Veröffentlichungstag der Anmeldung :
**09.12.92 Patentblatt 92/50**

(45) Bekanntmachung des Hinweises auf die
Patenterteilung :
**11.05.94 Patentblatt 94/19**

(84) Benannte Vertragsstaaten :
**AT BE CH DE DK ES FR GB GR IT LI LU NL SE**

(56) Entgegenhaltungen :
**DE-A- 3 828 576**
**TETRAHEDRON, vol.45, No.24,1989,**
**G.SCHNORRENBERG et al:"Fully Automatic**
**Simulataneous Multiple Peptide Synthesis in**
**Micromolar Scale-Rapid Synthesis of Series of**
**Peptides for Screening in BiologicalAssays",**
**pages 7759-7764, see whole document**

(56) Entgegenhaltungen :
**Proceedings OF THE NATIONAL ACADEMY**
**OF SCIENCES OF THE UNITED STATES OF**
**AMERICA, vol.82, no 15, August 1985, R-A.**
**Houghten " General metho for the rapid so-**
**lid-phase synthesis of large numbers of pepti-**
**des Specificity of antigen-antibody ineraction**
**at the level of individual amino acids" pages**
**5131-5135.see whole document**

(73) Patentinhaber : **BOEHRINGER INGELHEIM KG**
**D-55216 Ingelheim (DE)**
(84) **BE CH DE DK ES FR GR IT LI LU NL SE AT**
Patentinhaber : **BOEHRINGER INGELHEIM**
**INTERNATIONAL GmbH**
**D-55216 Ingelheim (DE)**
(84) **GB**

(72) Erfinder : **SCHNORRENBERG, Gerd**
**Ernst-Ludwig-Str. 66a**
**D-6535 Gau-Algesheim (DE)**
Erfinder : **KNAPP, Wilhelm**
**Winzerstr. 5**
**D-6537 Gensingen (DE)**

## Beschreibung

Für die schnelle Evaluierung von Struktur-Wirkungsbeziehungen an biologisch aktiven Peptiden durch Rezeptor-Bindungs-Studien und die schnelle Epitop-Ermittlung für die Immunologie bei Peptiden und Proteinen werden relativ kleine Mengen (unter je 20 mg) einer Vielzahl von Peptiden benötigt. Die Herstellung dieser Peptide erfolgt zweckmäßig nach der Festphasenpeptidsynthese. Diese Synthese basiert auf der von R.B. Merrifield entwickelten Methode (G. Barany, R.B. Merrifield in The Peptides, Analysis, Synthesis, Biology, Vol. 2, 3-284 (1980), Hrsg, Gross, Meienhofer Academic Press, New York), bei der die Peptidkette schrittweise aufgebaut wird. Die Syntheseschritte können wie folgt zusammengefaßt werden:

a) Binden der ersten Aminosäure der Peptidkette über eine Ankergruppe an einen polymeren Träger,
b) schrittweises Ankondensieren der übrigen Aminosäuren der Peptidkette,
c) Zwischenschritte zwischen den einzelnen Kondensationen bestehend aus Waschen, Abspalten von Schutzgruppen und Neutralisieren,
d) gewünschtenfalls acylieren endständiger Aminogruppen,
e) Abspalten des Peptids vom Träger.

Bei dieser Peptidsynthese muß mit einer Synthesezeit von bis zu 18 Stunden, meist bis zu 4 Stunden pro Aminosäure gerechnet werden. (Die einzelnen Kondensationen benötigen meist 1 bis 2 Stunden Reaktionszeit; zwischen den Kondensationen sind in der Regel etwa 10 Zwischenschritte erforderlich, für die je ca. 2 bis 15 Minuten gerechnet werden müssen.) Die Herstellung von Peptiden bestehend aus einer größeren Anzahl von Aminosäuren ist somit sehr langwierig, arbeitsintensiv und teuer.

Für die Festphasensynthese analoger Peptide ist von R. A. Houghten (Proc. Natl. Acad. Sci, USA, Vol. 82, pp. 5131-5135, August 1985, Immunology) eine Methode beschrieben worden. Danach wird der polymere Träger für die Synthese in Portionen von je 50-100 mg in kleine poröse Polypropylenbeutel gefüllt, die Beutel werden zugeschmolzen, die in den Synthesen einheitlichen Zwischenschritte (Waschen, Neutralisieren, Abspalten von Schutzgruppen) werden an allen Beuteln gleichzeitig in einem gemeinsamen Reaktionsgefäß ausgeführt, die einzelnen Kondensationen werden getrennt ausgeführt. Die Methode kann manuell oder teilweise automatisiert unter Verwendung eines Peptidsynthesizers ausgeführt werden.

Der Nachteil der beschriebenen Methode liegt darin, daß die Handhabung der Beutel etwas umständlich ist, daß die Beutel nicht wieder verwendet werden können, daß für die Kondensationen der verschiedenen Peptide die Beutel voneinander getrennt werden müssen und keine Kontrollproben während der ganzen Synthese entnommen werden können.

In der deutschen Patentanmeldung Nr. P 38 28 576.2 und in der Veröffentlichung G. Schnorrenberg und H. Gerhardt, Tetrahedron Vol. 45, No. 24, 7759-7764, 1989 wird ein Verfahren und eine Vorrichtung beschrieben, die die automatische simultane Synthese mehrerer Polypeptide ermöglicht und die oben genannten Nachteile vermeidet. Danach wird die bereits erwähnte Festphasensynthese-Methode so abgewandelt, daß sie mit Hilfe eines entsprechend angepaßten Pipettier-Roboters ausgeführt werden kann. Pipettier-Roboter sind bis jetzt für Serienanalysen verwendet worden. Zum Beispiel ist ein Pipettier-Roboter der Firma TECAN, RSP 5052, verwendbar. Pipettier-Roboter weisen folgende äußere Bestandteile auf: Mindestens einen Arm mit Dosierpipette, eine Halterung mit Vorratsgefäßen sowie eine Mikrotiterplatte die bis zu 96 Wells enthalten kann. Der Arm des Roboters bringt die Reagenzien aus den Vorratsgefäßen in die jeweiligen Wells der Microtiterplatte ein und saugt bei Bedarf Flüssigkeiten aus den Wells ab. Die Kanüle der Dosierpipette kann auch so ausgebildet sein, daß sie durch eine von oben nach unten laufende Trennwand in zwei Teile geteilt ist. (Durch diese geteilte Kanüle ist es möglich, zwei verschiedene Zudosierungen oder eine Zudosierung und eine Absaugung mit einem Arm auszuführen) Der Arbeitsablauf des Gerätes wird durch ein Computerprogramm gesteuert. Nach der genannten deutschen Patentanmeldung Nr. P 38 28 576.2 erfolgt die Festphasenpeptidsynthese in einem solchen Pipettier-Roboter wie folgt:

In den Wells einer Microtiterplatte wird Trägermaterial (vorzugsweise granuliertes Trägermaterial) vorgelegt. Das Trägermaterial kann mit dem Anfangsteil des jeweils gewünschten Peptids beladen sein. Die für die Reaktionen und Waschschritte benötigten Flüssigkeiten werden in den Vorratsgefäßen des Gerätes bereitgestellt. Soll am Ende der Synthese das Peptid vom Träger getrennt werden und/oder sollen freie Aminogruppen acyliert werden, so sind auch für diese Reaktionen die erforderlichen Reagenzien in den Vorratsgefäßen vorzubereiten. Aus den benötigten Reaktionszeiten ergibt sich, daß es zweckmäßig ist, eine Microtiterplatte zu verwenden, die nicht mehr als 96 Wells enthält. Dementsprechend können in einem Programmablauf maximal 96 verschiedene Polypeptide synthetisiert werden. Entsprechend dem Programm, das der Synthese dieser Peptide angepaßt worden ist, bringt der Roboter die Reagenzien und Waschflüssigkeiten in die einzelnen Wells ein und saugt nach der entsprechenden Verweilzeit die über dem Träger stehende Flüssigkeit jeweils ab. Anhand des zweiarmigen Pipettier-Roboters RSP 5052 der Firme TECAN wird das Verfahren und die nötige Anpassung des Gerätes an das Verfahren näher erläutert. Die Anwendung des Verfahrens ist jedoch nicht auf dieses Gerät beschränkt. Pipettier-Roboter anderer Bauart, insbesondere auch ein- oder mehrarmige Roboter können gemäß der vorliegenden Erfindung für das Verfahren angepaßt werden. Es wird eine Microtiterplatte mit 96 Wells gewählt. Ein Well faßt z. B. 10 mg Harz, das mit einer Aminosäure beladen sein kann, und etwas mehr als 300 µl Flüssigkeit. Diese Menge Harz entspricht etwa 5 µmol Aminosäure bzw. ist für die Herstellung von etwa 5 µmol Peptid geeignet. Übliche Trägermaterialien auf Polystyrol- oder Polyacrylamidbasis sind verwendbar. Es ist zweckmäßig, Peptide aufzubauen, die maximal 20 Aminosäuren enthalten. Die dafür benötigten Reagenzlösungen und Waschflüssigkeiten werden in den dafür vorgesehenen Vorratsgefäßen vorbereitet. Arm 1 des Gerätes ist mit einer Dosierpipette versehen, Arm 2 mit einer Absaugkanüle mit Spülvorrichtung. Diese Spülvorrichtung ist vorzugsweise mit einem separat stehenden Vorratsgefäß für das verwendete Lösungsmittel verbunden. Die Synthese erfolgt nach dem im angeschlossenen PC vorgegebenen Programm. Mit Arm 1 erfolgt die Zudosierung sämtlicher Reagenzlösungen, die aus offenen Vorratsgefäßen entnommen werden. Bevor die Dosierpipette von einer Reagenzlösung zu einer anderen wechselt, wird die Dosierpipette in einer speziellen Spülposition mit Lösungsmittel gespült. Mit Arm 2 erfolgt über eine mit einem Filter versehene Kanüle das Absaugen der Reagenz- und Waschflüssigkeiten. Zur Verhinderung von Harzverlusten und Kontaminationen der benachbarten Wells wird die Außenseite dieser Kanüle nach jedem Absaugvorgang mit Lösungsmittel über eine an der Kanüle seitlich angebrachte Zuleitung gespült. Mit diesem Lösungsmittel wird gleichzeitig der nächste Waschvorgang begonnen. Anschließend wird die Kanüle in einer Kanülenspülposition abgespült. Für die Abtrennung des Peptids von Harz wird z.B. Trifluoressigsäure über Arm 1 in die Wells eingebracht. Nach erfolgter Abspaltung wird mit der Absaugkanüle die Lösung abgesaugt und in eine zweite Mikrotiterplatte überführt, aus der dann die Aufarbeitung erfolgt.

Die beschriebene Vorrichtung ermöglicht die automatische simultane Synthese mehrerer Polypeptide. Es ist jedoch unbefriedigend, daß damit das Absaugen der Flüssigkeiten nicht vollständig erfolgen kann, und daß relativ viel Zeit für das Absaugen der Reagentien und Waschflüssigkeiten verwendet werden muß. Die vorliegende Erfindung überwindet die genannten Nachteile. Ferner ist ohne Wechsel der Reaktionsgefäße auch die Synthese von bis zu 25 µmol Peptid möglich.

Gegenstand der vorliegenden Erfindung ist eine Vorrichtung zur simultanen Synthese mehrerer Polypeptide nach der Festphasensynthesemethode, die mehrere Reaktionsgefäße und eine Haltevorrichtung für die Reaktionsgefäße aufweist, wobei die Reaktionsgefäße oben und unten offen sind, die untere Öffnung der einzelnen Reaktionsgefäße durch einen Filter abgedeckt ist, die Haltevorrichtung ein schließbares Gefäß ist, das eine Anschlußmöglichkeit für eine Inertgaszuleitung und eine Absaugvorrichtung aufweist sowie Öffnungen, in denen die Reaktionsgefäße so befestigt werden, daß deren obere Öffnungen von oben für das Zugeben der im Syntheseverfahren benötigten Flüssigkeiten zugänglich sind und deren untere Öffnungen mit dem Innenraum der Haltevorrichtung in Verbindung stehen.

Diese Vorrichtung kann in Verbindung mit zum Beispiel dem oben beschriebenen Pipettier-Roboter betrieben werden.

Die Erfindung betrifft ferner ein Verfahren zur simultanen Synthese mehrerer Polypeptide nach der Festphasensynthesemethode unter Verwendung der oben beschriebenen Vorrichtung, bei dem polymeres Trägermaterial oder mit der ersten Aminosäure oder einem Peptid beladenes polymeres Trägermaterial in den Reaktionsgefäßen vorgelegt wird, dann nach der an sich bekannten Festphasensynthesemethode in den Reaktionsgefäßen die Peptide aufgebaut werden und gewünschtenfalls freie Aminogruppen und/oder Hydroxygruppen der Peptide acyliert werden und/oder anschließend die Peptide von ihrem Trägermaterial abgetrennt werden, indem die für die einzelnen Schritte erforderlichen Reagenzien beziehungsweise Waschflüssigkeiten durch einen oder mehrere Roboterarm(e) mit Kanüle aus den entsprechenden Vorratsbehältern in die Reaktionsgefäße eingebracht werden und jeweils nach der benötigten Verweilzeit der Reagenzien beziehungsweise Waschflüssigkeiten die in den Reaktionsgefäßen oberhalb der Filter befindlichen Flüssigkeiten über die Haltevorrichtung gleichzeitig abgesaugt werden, wobei die einzelnen Schritte des Verfahrens durch das im Computer, der an den Roboter angeschlossen ist, vorgegebene Programm gesteuert werden. Eine bevorzugte Ausführung besteht darin, daß während des ganzen Syntheseverfahrens, ausgenommen sind nur die Phasen, in denen die Flüssigkeiten abgesaugt werden, Inertgas in die Haltevorrichtung eingeleitet wird, das unter so geringem Druck gehalten wird, daß es in den Reaktionsgefäßen nur das Durchsickern der Flüssigkeiten durch die Filter verhindert.

In einer bevorzugten Ausführungsform der Vorrichtung besteht die Haltevorrichtung aus einem wannenförmigen Gefäß mit plattenförmigem Deckel, wobei der Deckel Öffnungen aufweist, in denen je ein Reaktionsgefäß gehalten wird.

Geeignetes Material für die Haltevorrichtung und die Reaktionsgefäße ist z.B. Glas, Metall (vorzugsweise rostfreier Stahl), Polypropylen, Teflon oder Polyamid 66 oder eine Kombination dieser Materialien.

Die Haltevorrichtung und die Reaktionsgefäße müssen so aufeinander abgestimmt sein, daß die Reaktionsgefäße fest in der Haltevorrichtung ruhen. Das kann z.B. durch Einschrauben oder Einstecken der Reaktionsgefäße in die Haltevorrichtung erfolgen.

In einer bevorzugten Ausführungsform der Vorrichtung ist der Deckel der Haltevorrichtung mit entsprechenden konischen Öffnungen versehen und vorzugsweise aus Teflon oder Polyamid 66 hergestellt. Die Reaktionsgefäße sind zylinderische Glasbehälter, die in ihrem unteren Teil einen Glasschliff aufweisen, der in die Haltevorrichtung eingesteckt wird. Die Filter, die die unteren Öffnungen der Reaktionsgefäße abdecken, sind Glasfilterfritten oder Teflonfilterfritten. (Vorteilhaft ist es, Reaktionsgefäße zu verwenden, die im Boden je eine Bohrung aufweisen, in die die Teflonfilterfritte eingelegt beziehungsweise eingepreßt wird. Nach Beendigung eines Synthesecyclus können diese Fritten durch neue Fritten ersetzt werden.)

Wenn die erfindungsgemäße Vorrichtung mit dem genannten Pipettier-Roboter betrieben wird, ist es zweckmäßig die Vorrichtung mit maximal 48 Reaktionsgefäßen zu bauen.

Die Anwendung des Verfahrens ist jedoch nicht auf dieses Gerät beschränkt. Pipettier-Roboter anderer Bauart, insbesondere auch ein- oder mehrarmige Roboter können gemäß der vorliegenden Erfindung für das Verfahren angepaßt werden.

Für die Ausführung des erfindungsgemäßen Verfahrens wird in den Reaktionsgefäßen je z.B. 10-50 mg Harz vorgelegt. Diese Menge Harz entspricht etwa 5-25 μmol Aminosäure bzw. ist für die Herstellung von etwa 5-25 μmol Peptid geeignet. Übliche Trägermaterialien auf Polystyrol- oder Polyacrylamidbasis sind verwendbar. Es ist zweckmäßig, Peptide aufzubauen, die maximal 30 Aminosäuren enthalten. Die dafür benötigten Reagenzlösungen und Waschflüssigkeiten werden in den dafür vorgesehenen Vorratsgefäßen vorbereitet. Der Arm des Pipettier-Roboters ist mit einer Dosierpipette versehen. Die Synthese erfolgt nach dem im angeschlossenen PC vorgegebenen Programm. Damit erfolgt die Zudosierung sämtlicher Reagenzlösungen, die aus mit Teflon-Septen verschlosenen Vorratsgefäßen entnommen werden. Bevor die Dosierpipette von einer Reagenzlösung zu einer anderen wechselt, wird die Dosierpipette in einer speziellen Spülposition mit Lösungsmittel gespült. Analog werden die benötigten Waschflüssigkeiten eingebracht. Die Abspaltung des gewünschten Peptids vom Träger mit Trifluoressigsäure kann analog automatisch oder manuell ausgeführt werden.

Das Absaugen aller Flüssigkeiten aus den Reaktionsgefäßen der jeweiligen Umsetzung oder den Waschschritten erfolgt in allen Reaktionsgefäßen gleichzeitig durch die an die Haltevorrichtung angeschlossene Absaugvorrichtung (z.B. Wasserstrahlpumpe oder Membranpumpe).

Im allgemeinen wird DMF oder N-Methylpyrrolidon als Lösungsmittel eingesetzt. Dementsprechend müssen die Reaktionsgefäße und die eventuell vorgesehene Spülvorrichtung aus lösungsmittelbeständigem Material gefertigt sein, z.B. aus Glas, Polypropylen oder Teflon. In den handelsüblichen Geräten sind die Dosierpipetten aus Edelstahl gefertigt. Dieses Material ist für das erfindungsgemäße Verfahren geeignet.

Das Verfahren wird zum Beispiel mit folgenden Mitteln ausgeführt (Mengenangabe pro Well): Ausgangsmaterial ist 10 mg mit Fmoc-Aminosäuren beladenes Harz (Korngröße 200-400 mesh); Fmoc geschützte Aminosäuren werden in bis zu 10 fachem Überschuß für jeden einzelnen Kupplungsschritt eingesetzt, d.h. 200 μl einer DMF-Lösung von 50 μmol Fmoc-Aminosäure und 50 μmol 1-Hydroxybenzotriazol und 100 μl einer DMF-Lösung von 75 μmol N,N-Dicyclohexylcarbodiimid werden zugegeben; die Kupplungszeit beträgt ca. 1 Stunde. Die Abspaltung der Fmoc-Schutzgruppe erfolgt jeweils mittels 300 μl einer 40%igen Lösung von Piperidin in DMF. Die Abspaltungszeit ist ca. 20 Minuten. Die Waschschritte werden mit jeweils 300μl DMF ausgeführt.

Das Acylieren von freien Gruppen ($NH_2$, OH) kann analog durch Zugabe geeigneter Säureanhydride, z.B. Acetanhydrid und Pyridin, erfolgen. Nach Beendigung dieser Umsetzungen wird die Lösung abgesaugt und der Aufarbeitung zugeführt.

Die Abspaltung des fertigen Peptids vom Träger kann in den Reaktionsgefäßen erfolgen durch manuelle Zugabe von Trifluoressigsäure (20 Minuten Reaktionszeit). Die Peptide werden getrennt isoliert. Wie aus der obigen Erläuterung deutlich wird, werden alle Schritte der Peptidsynthese in offenen Gefäßen ausgeführt. Durch die erfindungsgemäße Ausführung der Synthese werden die Peptide trotzdem in sehr hoher Reinheit erhalten.

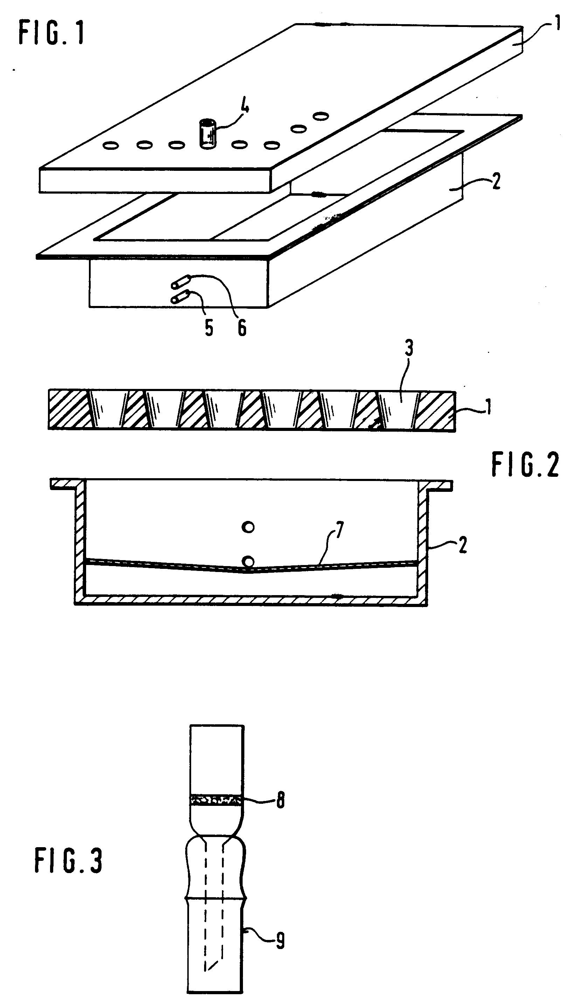

In den Figuren 1 bis 3 wird ein Beispiel der erfindungsgemäßen Vorrichtung schematisch gezeigt.

Figur 1 zeigt die Haltevorrichtung bestehend aus Wanne (2) und Deckel (1). Die Wanne (2) ist mit dem Deckel (1) fest verschließbar, vorzugsweise durch Schraubverschlüsse, die den Flansch der Wanne mit dem Deckel verbinden. Der Deckel (1) weist die Öffnungen (3) auf, die über die ganze Fläche in regelmäßigen Abständen angeordnet sind. (In der Zeichnung ist nur ein Teil der Öffnungen gezeichnet.) In diese Öffnungen werden die Reaktionsgläser (4) gesteckt. Falls das Verfahren nur in weniger Reaktionsgefäßen ausgeführt wird als der Deckel Öffnungen aufweist, werden die unbenutzten Öffnungen durch Schliffstopfen verschlossen.

In der Wand der Wanne (2) sind 2 Anschlüsse (5) und (6) vorgesehen. Anschluß 5 ist an die Absaugvorrichtung angeschlossen, Anschluß 6 dient der Zufuhr von Inertgas.

Figur 2 zeigt den Querschnitt einer bevorzugten Ausführungsform der Haltevorrichtung. Diese enthält ein Leitblech 7, das wie ein zweiter Boden in die Wanne (2) knapp unterhalb des Anschlusses (5) angebracht ist.

Es ist leicht abfallend zum Anschluß (5) hin angeordnet, so daß restloses Abziehen der abgesaugten Flüssigkeiten ermöglicht wird.

Figur 3 zeigt die schematische Darstellung eines Reaktionsgefäßes (4). Der obere Teil des Gefäßes ist zylindrisch. Der untere Teil des Gefäßes verjüngt sich und ist mit einem Schliff (9) fest verbunden. Die untere Öffnung des Reaktionsgefäßes ist durch eine Glasfilterfritte oder Teflonfilterfritte (8) abgedeckt.

In diesem Ausführungsbeispiel ist die Wanne (2) aus Metall (vorzugsweise V2a-Stahl), der Deckel (1) aus Teflon oder Polyamid 66, und die Reaktionsgefäße (4) aus Glas. Die Öffnungen (3) sind konisch und entsprechen genau der Größe der Schliffe (9) der Reaktionsgefäße. Die Filter (8) sind Glasfilterfritten G2 oder G3 oder Teflonfilterfritten der Firma G.T. Baker Chemikalien, DE-6080 Groß-Gerau, Bestellnummer 7329/03. Wenn die Vorrichtung zusammen mit dem Pipettierroboter der Firma TECAN, RSP 5052, verwendet wird, ist es zweckmäßig die Wanne (2) mit etwa den Maßen 165x127x45 (mm) herzustellen. Die Reaktionsgefäße haben dann eine Gesamthöhe von etwa 70 mm, von der etwa 25 mm auf den zylindrischen Teil oberhalb der Glasfilterfritte entfallen. Der Durchmesser dieses oberen Teils beträgt 13 mm. Es ist zweckmäßig die Vorrichtung für maximal 48 Reaktionsgefäße zu bauen.

Die Haltevorrichtung ist über den Anschluß 5 mit einer Absaugvorrichtung, z.B. einer Membranpumpe mit vorgeschalteter 5 l-Saugflasche verbunden. Ferner ist die Haltevorrichtung über den Anschluß 6 mit einer Inertgaszufuhr (z.B. Stickstoffflasche) verbunden. In dieser Leitung ist ein Überdruckventil (meist eingestellt auf etwa 0,1 bar) vorgesehen. Außerdem sind über z.B. einen PC-steuerbare Regelorgane in den Leitungen vorgesehen.

Eine Variante dieser Vorrichtung ist so gestaltet, daß die Wanne (2) nur einen Anschluß aufweist, der über ein PC-steuerbares Dreiwegventil mit den beiden Leitungen (Inertgas/Absaugvorrichtung) verbunden ist.

Das folgende Beispiel erläutert den Ablauf des erfindungsgemäßen Verfahrens: Dabei wird die oben beschriebene Vorrichtung in Verbindung mit einem Pipettier-Roboter verwendet. Der Ablauf des Verfahrens ist computergesteuert. Trägermaterial, das bereits mit einer geschützten Aminosäure oder einem kurzen Peptid beladen ist, wird in den Reaktionsgefäßen vorgelegt.

Teil des Synthesecyclus:

Schritt                                              Vorgang

1     Ventil $N_2$ auf, Vakuum zu

2     Zudosieren von DMF 3 Min.                      Waschen

3     Ventil $N_2$ zu, Vakuum auf                    Absaugen

4     Ventil $N_2$ auf, Vakuum zu

5     Zudosieren 40 % Piperidin im DMF               Schutzgruppen-
      3 Min.                                         Abspaltung

6     Ventil $N_2$ zu, Vakuum auf                    Absaugen

7     Ventil $N_2$ auf, Vakuum zu

8     Zudosieren 40 % Piperidin im DMF               Schutzgruppen-
      20 Min.                                        Abspaltung

9     Ventil $N_2$ zu, Vakuum auf                    Absaugen

10    Ventil $N_2$ auf, Vakuum zu

11    Zudosieren von DMF 30 Sek.                     Waschen

12    Ventil $N_2$ zu, Vakuum auf                    Absaugen

13    Ventil $N_2$ auf, Vakuum zu

14-40 Wiederholung Schritte 11-13                    Waschen 9x

41    Zudosieren der gewünschten
      Fmoc-Aminosäure/HOBt in DMF                    1. Kupplung

42    Zudosieren DIC/DMF 40 Min.                     1. Kupplung

43    Ventil $N_2$ zu, Vakuum auf                    Absaugen

44    Ventil $N_2$ auf, Vakuum zu

45-48 Wiederholung Schritte 41-44                    2. Kupplung

49-78 Wiederholung Schritte 11-13                    Waschen 10x

Veglichen mit dem Verfahren und der Vorrichtung gemäß der genannten deutschen Patentanmeldung Nr. P 38 28 576.2 ermöglicht die erfindungsgemäße Vorrichtung eine wesentliche Verkürzung (etwa Halbieren) der für den Synthesecyclus benötigten Zeit. Die Synthese erfolgt verläßlich und sauber, da die Absaugung der Flüssigkeiten vollständig erfolgt. Der Ablauf der Synthese kann leicht kontrolliert werden, da die Reaktionsgefäße offen sind.

**Patentansprüche**

1.  Vorrichtung zur simultanen Synthese mehrerer Polypeptide nach der Festphasensynthesemethode, die mehrere Reaktionsgefäße und eine Haltevorrichtung für die Reaktionsgefäße aufweist, wobei die Reaktionsgefäße oben und unten offen sind, die untere Öffnung der einzelnen Reaktionsgefäße durch einen Filter abgedeckt ist, die Haltevorrichtung ein schließbares Gefäß ist, das eine Anschlußmöglichkeit für eine Inertgaszuleitung und eine Absaugvorrichtung aufweist sowie Öffnungen, in denen die Reaktionsgefäße so befestigt werden, daß deren obere Öffnungen von oben für das Zugeben der im Syntheseverfahren benötigten Flüssigkeiten zugänglich sind und deren untere Öffnungen mit dem Innenraum der Haltevorrichtung in Verbindung stehen.

2. Vorrichtung nach Anspruch 1, dadurch gekennzeichnet, daß die Haltevorrichtung aus einem wannenförmigen Gefäß mit plattenförmigem Deckel besteht, wobei der Deckel Öffnungen aufweist, in denen je ein Reaktionsgefäß gehalten wird.

3. Vorrichtung nach Anspruch 1 oder 2, dadurch gekennzeichnet, daß die Reaktionsgefäße zylinderische Glasbehälter sind, die in ihrem unteren Teil einen Glasschliff aufweisen, der in die Haltevorrichtung eingesteckt wird.

4. Vorrichtung nach einem der Ansprüche 1 bis 3, dadurch gekennzeichnet, daß die Filter, die die unteren Öffnungen der Reaktionsgefäße abdecken, Glasfilterfritten oder Teflonfilterfritten sind.

5. Vorrichtung nach einem der Ansprüche 1 bis 4, dadurch gekennzeichnet, daß die für das Syntheseverfahren benötigten Flüssigkeiten durch einen Pipettier-Roboter in die Reaktionsgefäße eingebracht werden.

6. Verfahren zur simultanen Synthese mehrerer Polypeptide nach der Festphasensynthesemethode unter Verwendung der Vorrichtung nach einem der Ansprüche 1 bis 5, bei dem polymeres Trägermaterial oder mit der ersten Aminosäure oder einem Peptid beladenes polymeres Trägermaterial in den Reaktionsgefäßen vorgelegt wird, dann nach der an sich bekannten Festphasensynthesemethode in den Reaktionsgefäßen die Peptide aufgebaut werden und gewünschtenfalls freie Aminogruppen und/oder Hydroxygruppen der Peptide acyliert werden, indem die für die einzelnen Schritte erforderlichen Reagenzien beziehungsweise Waschflüssigkeiten durch einen oder mehrere Roboterarm(e) mit Kanüle aus den entsprechenden Vorratsbehältern in die Reaktionsgefäße eingebracht werden und jeweils nach der benötigten Verweilzeit der Reagenzien beziehungsweise Waschflüssigkeiten die in den Reaktionsgefäße oberhalb der Filter befindlichen Flüssigkeiten über die Haltevorrichtung gleichzeitig abgesaugt werden,
wobei die einzelnen Schritte des Verfahrens durch das im Computer, der an den Roboter angeschlossen ist, vorgegebene Programm gesteuert werden.

7. Verfahren nach Anspruch 6, dadurch gekennzeichnet, daß während des ganzen Syntheseverfahrens, ausgenommen sind nur die Phasen, in denen die Flüssigkeiten abgesaugt werden, Inertgas in die Haltevorrichtung eingeleitet wird, das unter so geringem Druck gehalten wird, daß es in den Reaktionsgefäßen das Durchsickern der Flüssigkeiten durch die Filter verhindert.

## Claims

1. Apparatus for simultaneously synthesising a plurality of polypeptides by the solid phase synthesis method, comprising a plurality of reaction vessels and a retaining device for the reaction vessels which are open at the top and bottom, the bottom opening of the individual reaction vessels being covered by a filter, the retaining device being a closable vessel which has a possible connection for an inert gas supply line and a suction device, as well as openings in which the reaction vessels are fixed in such a way that the upper openings thereof are accessible from above for the addition of the liquids required in the synthesis process whilst the bottom openings thereof communicate with the interior of the retaining device.

2. Apparatus according to claim 1, characterised in that the retaining device consists of a tub-shaped vessel with a plate-shaped lid, the lid having openings each of which contains a reaction vessel.

3. Apparatus according to claim 1 or 2, characterised in that the reaction vessels are cylindrical glass containers which have, on their underside a ground glass portion which is inserted in the retaining device.

4. Apparatus according to one of claims 1 to 3, characterised in that the filters which cover the bottom openings of the reaction vessels are glass filter frits or Teflon filter frits.

5. Apparatus according to one of claims 1 to 4, characterised in that the liquids required for the synthetic process are introduced into the reaction vessels by means of a pipetting robot.

6. Process for simultaneously synthesising a plurality of polypeptides by the solid phase synthesis method

7

using the apparatus according to one of claims 1 to 5, wherein polymeric carrier material or polymeric carrier material charged with the first amino acid or a peptide is placed in the reaction vessels, then using a known solid phase synthesis method the peptides are synthesised in the reaction vessels and, if desired, free amino groups and/or hydroxy groups of the peptides are acylated, by introducing the reagents or washing liquids required for the individual steps into the reaction vessels from the appropriate storage containers by means of one or more robot arm(s) with cannulas, and after the necessary retention time for the reagents or washing liquids the liquids contained in the reaction vessels above the filters are simultaneously sucked out via the retaining device, whilst the individual steps of the process can be controlled by the program set up in the computer connected to the robot.

7. Process according to claim 6, characterised in that, throughout the entire process of synthesis, with the sole exception of the phases in which the liquids are sucked out, inert gas is introduced into the retaining device, this gas being maintained under such slight pressure that in the reaction vessels it prevents the liquids from trickling through the filters.

**Revendications**

1. Dispositif de synthèse simultanée de plusieurs polypeptides selon la méthode de synthèse en phase solide, qui comporte plusieurs récipients réactionnels et un dispositif dé maintien pour les récipients réactionnels, les récipients réactionnels étant ouverts en haut et en bas, l'ouverture inférieure des différents récipients réactionnels étant recouverte par un filtre, le dispositif de maintien étant un récipient fermable qui présente une possibilité de raccordement pour une conduite d'amenée de gaz inerte et un dispositif d'aspiration ainsi que des ouvertures dans lesquelles les récipients réactionnels sont fixés de manière que leurs ouvertures supérieures soient accessibles par en haut pour l'introduction des liquides nécessaires dans le procédé de synthèse et que leurs ouvertures inférieures soient en communication avec la cavité interne du dispositif de maintien.

2. Dispositif selon la revendication 1, caractérisé en ce que le dispositif de maintien consiste en un récipient en forme de cuve à couvercle en forme de plaque, le couvercle comportant des ouvertures dans chacune desquelles est maintenu un récipient réactionnel.

3. Dispositif selon la revendication 1 ou 2, caractérisé en ce que les récipients réactionnels sont des récipients en verre cylindriques qui comportent à leur partie inférieure un rodage en verre qui est inséré dans le dispositif de maintien.

4. Dispositif selon l'une des revendications 1 à 3, caractérisé en ce que les filtres qui recouvrent les ouvertures inférieures des récipients réactionnels sont des frittes filtrantes en verre ou des frittes filtrantes en Teflon.

5. Dispositif selon l'une des revendications 1 à 4, caractérisé en ce que les liquides nécessaires pour le procédé de synthèse sont introduits par un robot de pipettage dans les récipients réactionnels.

6. Procédé de synthèse simultanée de plusieurs polypeptides selon la méthode de synthèse en phase solide au moyen du dispositif selon l'une des revendications 1 à 5, dans lequel un matériau support polymérique ou un matériau support polymérique sur lequel est fixé le premier acide aminé ou un peptide est disposé au préalable dans les récipients réactionnels, puis les peptides sont formés dans les récipients réactionnels selon la méthode de synthèse en phase solide connue en soi et, si on le souhaite, les groupes amine et/ou hydroxyle libres des peptides sont acylés par le fait que les réactifs ou les liquides de lavage nécessaires pour les différentes étapes sont introduits depuis les récipients de stockage correspondants dans les récipients réactionnels par un ou plusieurs bras de robot munis d'une canule et, après le temps de séjour nécessaire des réactifs ou des liquides de lavage, les liquides qui se trouvent dans les récipients réactionnels au dessus des filtres sont aspirés simultanément par l'intermédiaire du dispositif de maintien, les différentes étapes du procédé étant commandées par le programme préétabli dans l'ordinateur qui est raccordé aux robots.

7. Procédé selon la revendication 6, caractérisé en ce que, pendant tout le procédé de synthèse, à l'exception seulement des phases dans lesquelles les liquides sont aspirés, un gaz inerte, qui est maintenu sous une pression si faible qu'il empêche dans les récipients réactionnels le passage des liquides à travers les filtres, est introduit dans le dispositif de maintien.

FIG.1

FIG.2

FIG.3